# EUROPÄISCHE PATENTSCHRIFT

(11) **EP 1 757 274 B1**
(45) Veröffentlichungstag und Bekanntmachung des Hinweises auf die Patenterteilung: **25.07.2012**
(21) Anmeldenummer: 05017201.4
(22) Anmeldetag: 08.08.2005
(51) Int. Cl.: A61K 9/46, A61K 31/167, A61K 31/522, A61K 31/4402, A61K 31/375

(54) **Brausezubereitung gegen Erkältungssymptome**
Effervescent composition against cold symptoms
Composition effervescente contre les symptomes du rhume

(43) Veröffentlichungstag der Anmeldung: 28.02.2007
(73) Patentinhaber: STADA ARZNEIMITTEL AG, 61118 Bad Vilbel (DE)
(72) Erfinder: Hansen, Peter Dr., 63303 Dreieich (DE)
(74) Vertreter: Hamm, Volker

(56) Entgegenhaltungen:
- DE-A1- 19 502 789
- US-A- 3 882 228
- US-A- 4 466 960
- US-A1- 2004 029 864
- "ROTE LISTE 2002, Arzneimittelverzeichnis für Deutschland" 2002, EDITIO CANTOR VERLAG , AULENDORF , XP002357950 Kennziffer 05 462

## Beschreibung

Die vorliegende Erfindung betrifft eine klarlösende Brausezubereitung zur Behandlung von Erkältungssymptomen, die eine Kombination aus einem Analgetikum, einem Antihistaminikum, Coffein und Ascorbinsäure umfasst.

Brausetabletten oder -granulate sind nicht überzogene Tabletten oder Granulate, die üblicherweise aus einem Wirkstoff, einem Brausesatz und gegebenenfalls aus weiteren Hilfsstoffen bestehen. Der Brausesatz besteht aus einer Säurekomponente (wie zum Beispiel Citronen- oder Weinsäure) und einer basischen Komponente (wie zum Beispiel Carbonate oder Hydrogencarbonate), bei deren Reaktion miteinander rasch Kohlendioxid freigesetzt wird.

Brausezubereitungen, wie z. B. Brausepulver oder Brausetabletten, werden als Formulierungsform beispielsweise bei Wirkstoffen, die eine lange Resorptionszeit oder Magenschleimhautreizende Eigenschaften aufweisen, eingesetzt. Ferner werden Brausetabletten besonders gern dann eingesetzt, wenn ein schneller Wirkungseintritt erwünscht ist, wie zum Beispiel bei Erkältungssymptomen oder Schmerzen.

Gerade bei Erkältungen, die mehrere Symptome umfassen können, oder auch bei Schmerzen werden bereits seit langem Kombinationspräparate am Patienten eingesetzt. Vorteile eines Kombinationspräparates liegen vor allem in der fixen Kombination mehrerer Wirkstoffe in nur einer Zubereitungsform, sowie gegebenenfalls in deren synergistischen Wirkung. Diesbezüglich gängige Wirkstoffkombinationen sind zum Beispiel Paracetamol/Coffein, Acetylsalicylsäure/Coffein oder Acetylsalicylsäure/Ascorbinsäure.

Paracetamol ist ein schmerzstillender und fiebersenkender Wirkstoff. Er hemmt die Produktion von Prostaglandinen, wichtigen Botenstoffen bei Schmerz- und Entzündungsreaktionen. Acetylsalicylsäure (ASS) ist ein schmerzstillender, fiebersenkender und entzündungshemmender Wirkstoff, welcher ebenfalls in die Prostaglandinsynthese eingreift. Coffein verstärkt die Wirkung und den Wirkungseintritt anderer Wirkstoffe, wie zum Beispiel von Paracetamol. Ferner regt Coffein den Herzkreislauf an, wobei Coffein Gefäße von Haut, Niere und Herz erweitert werden. Der kortikale Effekt des Coffeins führt vorrübergehend zur Erhöhung der Leistungsfähigkeit; die Müdigkeit verschwindet, die geistige Aufnahmefähigkeit, das Merkvermögen und die Denkfähigkeit werden gesteigert. Ascorbinsäure, eine andere Bezeichnung für Vitamin C, ist im Körper am Aufbau vom Bindegewebe, an der Bildung von Botenstoffen und beim Abbau von Fremd- und Wirkstoffen beteiligt. Ferner spielt Ascorbinsäure eine Rolle bei der Immunabwehr; eine ausreichende Menge an Ascorbinsäure kann den Widerstand gegen Infektionskrankheiten erhöhen. Kombinationspräparate bestehend aus einer zweier oder dreier Kombination dieser Wirkstoffe sind aufgrund ihrer synergistischen und ergänzenden Wirkungen sinnvoll und bereits bekannt.

Brausetabletten zur Behandlung von Erkältungssymptomen und/oder Schmerzen, die eine Kombination von zwei oder drei Wirkstoffen enthalten, sind bekannt; sie umfassen ASS (Acetylsalicylsäure) und Vitamin C, ASS, Paracetamol und Vitamin C oder ASS, Vitamin C und Coffein.

US 2004/0029864 offenbart eine Zubereitung gegen Erkältungssymptome enthaltend Chlorphenamin, Paracetamol, Coffein und Ascorbinsäure. Die Anmeldung offenbart weder eine konkrete Galenik für die vorstehend genannte Vierer-Kombination noch die Möglichkeit, Medikamente gegen Erkältungssymptome als Brausezubereitungen zu formulieren.

Eine unter dem Handelsnamen Togal^{®} Kopfschmerz-Brausetablette enthaltend Acetylsalicylsäure, Ascorbinsäure und Coffein wurde am Anmeldetag vermarktet (Rote Liste^{®} 2002)

DE 195 02 789 beschreibt eine Paracetamol, Acetylsalicylsäure und Coffein enthaltende Brausezubereitung.

Eine Kombination eines Analgetikums, eines Antihistaminikums, Coffein und Ascorbinsäure in Form einer Brausetablette, ist bisher nicht bekannt. Dies ist vermutlich darauf zurückzuführen, dass erhebliche Schwierigkeiten bei der Herstellung solcher Wirkstoffkombination in Form von Brausetabletten auftreten.

Die Zusammensetzung von Tabletten muss individuell für jeden Wirkstoff, für jeden Verwendungszweck und für jede Herstellungstechnologie entwickelt werden. Jeder Wirkstoff (und auch jeder Hilfsstoff) hat bestimmte physikalische und chemische Eigenschaften, z. B. gute oder schlechte Komprimierbarkeit oder Löslichkeit, höhere oder niedrigere Hygroskopizität, Hydrolyse- oder Oxidationsempfindlichkeit etc. Diese Eigenschaften sind bei der Wahl der Hilfsstoffe und der Herstellungsverfahren genauso zu berücksichtigen, wie die angestrebte Bioverfügbarkeit und Haltbarkeit der fertigen Arzneiform.

Im besonderen Fall der Formulierung von Kombinationspräparaten, entstehen basierend auf den unterschiedlichen chemischen und physikalischen Eigenschaften der Ausgangsstoffe nicht vorhersehbare Wechselwirkungen nicht nur zwischen Wirkstoffen und Hilfsstoffen, sondern vielmehr auch zwischen den Wirkstoffen untereinander. Insbesondere treten Homogenitätsprobleme auf, wenn einer dieser enthaltenen Wirkstoffe gegenüber den anderen Wirkstoffen in relativ geringen Mengen enthalten ist.

Wechselwirkungen unter den Ausgangsstoffen können bekannter Weise u.a. zur Verfärbungen, Gasentwicklung, Wirkungsverlust, unerwünschte Wirkungssteigerung, verzögerte Freigabe des Wirkstoffs etc. führen. Weitere Inkompatibilitäten während der Tablettenherstellung umfassen u. a. die Bildung von eutektischen Gemischen unter Verflüssigung bei Einsatz von zwei oder mehr kristallinen Stoffen mit im allgemeinen niedrigen Schmelzbereichen; dies tritt zum Beispiel beim Verreiben, Trocknen oder Tablettieren auf. Ferner kann es, aufgrund stark divergierender pulvertechnologischer Eigenschaften (wie zum Beispiel Pulvergrößenverteilung, Partikelform etc.), zur Entmischung von Pulvern während der Verarbeitung zu Tabletten kommen.

Zusätzlich zu den zu erwartenden Inkompatibilitäten/Instabilitäten bei der Herstellung eines Kombinationspräparats, erweisen sich die besonderen Eigenschaften einer Brausezubereitung im Herstellungsverfahren als problematisch. Eine Brausezubereitung umfasst üblicherweise eine Säurekomponente und eine basische Komponente als CO₂-Spender, womit der Fachmann an den Zusatz bestimmter Hilfsstoffe gebunden ist. Zusätzlich muss sich die Brausezubereitung innerhalb weniger Minuten in Wasser auflösen und eine stabile Wirkstofflösung bilden. Des weiteren handelt es sich bei der Brausezubereitung um eine äußerst wasserempfindliche Darreichungsform; im Falle einer Feuchtgranulierung, darf die Granulierflüssigkeit kein Wasser enthalten.

Brausegranulate werden üblicherweise in Form von Krustengranulaten hergestellt. Bei der Herstellung von Brausetabletten, werden die Komponenten des Brausesatzes, ggf. in Kombination mit einem Wirkstoff, vor der Tablettierung einem Granulationsverfahren unterworfen, da sowohl der CO₂-Spender als auch die saure Komponente für eine Direkttablettierung schlecht geeignet sind. Üblicherweise werden Brausetabletten in 3 bis 4 Stufen hergestellt, nämlich durch Granulation des Brausesatzes aus CO₂-Spender und CO₂-freisetzender saurer Komponente, Mischen der üblichen Komponenten (Wirkstoffe und weitere Hilfsstoffe), Vereinigung der aus den ersten Verfahrensschritten erhaltenen Komponenten und Tablettierung der erhaltenen Mischung. Auch besondere Verfahren bezüglich einer Direkttablettierung von Brausetabletten sind aus dem Stand der Technik bekannt, z. B. aus DE 44 20 735A oder DE 40 27 927A.

Aufgabe der vorliegenden Erfindung ist es damit, stabile, schnell- und klarlösende Brausezubereitungen anzugeben, die vier verschiedene Wirkstoffe aus der Gruppe der Analgetika, der Gruppe der Antihistaminika, Coffein und Ascorbinsäure enthalten.

Insbesondere ist es eine Aufgabe der vorliegenden Erfindung, eine Zusammensetzung anzugeben, in der ein niedrig dosierter Wirkstoff in der klargelösten Brausezubereitung homogen verteilt ist und die Brauselösung gleichzeitig einen guten Geschmack aufweist.

Gelöst wird diese Aufgabe durch die Merkmale des unabhängigen Anspruchs. Die abhängigen Ansprüche definieren vorteilhafte Ausführungsformen der Erfindung.

In den der vorliegenden Erfindung zugrundeliegenden Untersuchungen wurde überraschend gefunden, dass, wenn man eine Wirkstoffkombination bestehend aus einem Analgetikum, einem Antihistaminikum, das aus Chlorphenamin Doxylamin Diphenylpyralin und desen Salze und Hydrate ausgwählt ist, Coffein und Ascorbinsäure, mit anderen Hilfsstoffen und/oder ggf. mit Geschmackskorrigenzien mischt, granuliert, und ggf. in an sich bekannter Weise tablertiert, stabile Brausezubereitungen in Form von Granulaten oder Tabletten erhalten werden. Die vorliegenden Brausezubereitungen lösen sich zudem schnell auf, ergeben klare, gut schmeckende Lösungen, in denen die Wirkstoffe homogen verteilt sind.

Der hierin benutzte Ausdruck "stabile Brausezubereitung" bezieht sich auf eine Brausezubereitung die solange als stabil zu bezeichnen ist, wie das Ausmaß der Veränderungen den festgelegten Schwellenwert der Spezifikationen (Wirkstoffgehalt unmittelbar nach Herstellung ± 5% der Deklaration; während der Laufzeit ≥ 90% der Deklaration bzw. entsprechend der u.a. EG-Richtlinien; gesicherte sensorisch wahrnehmbare, physikalisch-chemische und mikrobiologische Eigenschaften) nicht überschreitet.

Erfindungsgemäß bevorzugte Wirkstoffe umfassen Analgetika wie Paracetamol, Acetylsalicylsäure, Ibuprofen, Ketoprofen, Meloxicam, Naproxen, Prophyphenazon, Metamizol, sowie deren Salze/Hydrate und H₁-Antihistaminika wie Chlorphenaminmaliert, Doxylaminsuccinat und Diphenylpyralin- hydrochlorid. Besonders bevorzugt eingesetzt werden Paracetamol und Acetylsalicylsäure sowie Chlorphenaminmaleat.

Als Säurekomponenten, die der Freisetzung von Kohlendioxid aus dem CO₂-Spender dient, enthalten die erfindungsgemäßen Zusammensetzungen physiologisch unbedenkliche Säuren (sogenannte "Genusssäuren"), die stark genug sind, Kohlendioxid aus dem CO₂-Spender freizusetzen, wie z. B. Citronensäure, Ascorbinsäure, Äpfelsäure, Fumarsäure, Weinsäure, Bernsteinsäure, Maleinsäure, Malonsäure, Adipinsäure, Mononatriumcitrat, Dinatriumcitrat, Kaliumhydrogentartrat, oder Natriumhydrogenphosphat sowie deren Mischungen. Bevorzugt eingesetzt werden Citronensäure oder Weinsäure.

Geeignete CO₂-Spender sind Alkali- und Erdalkalicarbonate und -hydrogencarbonate, insbesondere Natrium- und Kaliumcarbonat, Natrium- und Kaliumhydrogencarbonat sowie Magnesium- und Calciumcarbonat.

Die erfindungsgemäßen Zusammensetzungen enthalten des weiteren übliche Hilfsstoffe, wie Füllstoffe (z.B. eine wasserlösliche, nicht schäumende Komponente wie Saccharose, Lactose, Mannitol, Mononatriumcitrat, oder eine brausende Komponente, wie z. B. Natrium- oder Kaliumhydrogencarbonat, Natriumcarbonat, Kaliumcarbonat oder Mischungen dieser Komponenten), Bindemittel (z.B. Polyvidon), Fließreguliermittel, Sprengmittel, Schmiermittel (wie z. B. Stearinsäure), Zuckeralkohole (wie z. B. Sorbitol, Mannitol, oder ähnliche), PEG, Süßstoffe (wie z. B. Saccharin-Natrium, Aspartam, Acesulfam-Kalium, Natrium-Cyclamat), und/oder Aromen (wie z. B. Citronenaroma oder andere Fruchtaromen).

In einer bevorzugten Ausführungsform der erfindungsgemäßen Zusammensetzungen wird zunächst der Brausesatz granuliert. Parallel dazu werden Paracetamol, Chlorphenaminmaleat, Coffein, Ascorbinsäure mit ggf. weiteren Hilfsstoffen vermischt, wobei Chlorphenaminmaleat in einer Menge unterhalb von 10 mg pro Dosiseinheit enthalten ist. Anschliessend wird das Wirkstoff-Hilfsstoff-Gemisch mit dem den Brausesatz enthaltenem Granulat vereinigt, vermischt und tablettiert.

Vorzugsweise enthält jede Einzeldosis der Brausezubereitung eine wirksame Menge an Paracetamol zwischen 200 bis 500 mg, an Coffein zwischen 25 und 75 mg, an Chlorphenaminmaleat zwischen 2,5 bis 10 mg und an Ascorbinsäure zwischen 100 bis 300 mg.

Gemäß einer weiteren bevorzugten Ausführungsform wird der Brausesatz mit mindestens einem der vier Wirkstoffe granuliert, und anschließend mit einem Wirkstoff-Hilfsstoff-gemisch oder einem Hilfsstoffgemisch vermengt, vermischt und tablettiert.

Gemäß einer weiteren bevorzugten Ausführungsform wird eine Komponente oder beide Komponenten des Brausesatzes ggf. mit einem oder mehreren Wirkstoffen getrennt voneinander granuliert und anschließend mit einem Wirkstoff-Hilfsstoffgemisch oder einem Hilfsstoffgemisch vermengt, vermischt und tablettiert.

Nach einer weiteren bevorzugten Ausführungsform werden der Brausesatz, die vier Wirkstoffe und ggf. weitere Hilfsstoffe und/oder Geschmackskorrigenzien direkt tablettiert.

Gemäß einer weiteren bevorzugten Ausführungsform liegt die erfindungsgemäße Zusammensetzung in Form von Krustengranulaten vor, welche ggf. zu Tabletten weiterverarbeitet werden können.

Der Vorteil der erfindungsgemäßen Zusammensetzung liegt vor allem darin, dass die stabile Vierer-Kombination in Form einer Brausetablette oder eines Brausegranulats mehrere Symptome einer Erkältung, wie z.B. Kopf- und Gliederschmerzen, Rhinitis, Schlappheit etc. gleichzeitig bekämpft. Zudem ergibt die Brausezubereitung eine klare, gutschmeckende Lösung, die dem Patienten die Einnahme erleichtert und dabei eine schnelle Linderung der Symptome durch einen schnellen Wirkungseintritt ermöglicht. Ein weiterer Vorteil liegt in den zusätzlich zu ihren eigentlichen Wirkungsspektren synergistischen Effekten des Coffeins und der Ascorbinsäure.

Die nachfolgenden Beispiele sollen die Erfindung näher erläutern ohne sie einzuschränken.

Die nachfolgenden Ausführungsbeispiele 1-2 beschreiben exemplarisch die Zusammensetzung einer erfindungsgemäßen Brausetablette, gemäß eines herkömmlichen Herstellungsprozesses von Brausetabletten, basierend auf einer vorangehenden Granulation des Brausesatzes aus CO₂-Spender und CO₂-freisetzender saurer Komponente, Mischen der Wirkstoffe mit den übrigen Hilfsstoffen, Vereinigung der aus den ersten Verfahrensschritten erhaltenen Komponenten und Tablettierung der resultierenden Mischung.

### Beispiel 1

| | |
|---|---|
| Paracetamol | 300 mg |
| Coffein | 50 mg |
| Chlorphenaminmaleat | 5 mg |
| Vitamin C | 200 mg |
| NaHCO₃ | 400 mg |
| Weinsäure | 500 mg |
| Polyvidon | 100 mg |
| Lactose-Monohydrat | 95 mg |
| Sorbit | 95 mg |
| Saccharin-Natrium | 10 mg |
| Orangenaroma | 50 mg |

### Beispiel 2

| | |
|---|---|
| Paracetamol | 400 mg |
| Coffein | 50 mg |
| Chlorphenaminmaleat | 5 mg |
| Vitamin C | 300 mg |
| NaHCO₃ | 1000 |
| Citronensäure | 1200 |
| Polyvidon | 192 mg |
| Lactose-Monohydrat | 120 mg |
| Mannit | 120 mg |
| Mg-Stearat | 90 mg |
| Saccharin-Natrium | 15 mg |
| Citronenaroma | 55 mg |

Das nachfolgende Ausführungsbeispiel 3 beschreibt exemplarisch die erfindungsgemäße Zusammensetzung einer Brausetablette, gemäß eines herkömmlichen Herstellungsprozesses von Brausetabletten, basierend auf einer vorangehenden, getrennten Granulation der CO₂-spendenden basischen und CO₂-freisetzenden sauren Komponente des Brausesatzes, mischen der Wirkstoffe mit den übrigen Hilfsstoffen, Vereinigung der aus den ersten Verfahrensschritten erhaltenen Komponenten und Tablettierung der daraus resultierenden Mischung.

### Beispiel 3

| | |
|---|---|
| Paracetamol | 500 mg |
| Coffein | 75 mg |
| Chlorphenaminmaleat | 5 mg |
| Vitamin C | 250 mg |
| NaHCO₃ | 240 mg |
| Citronensäure | 300 mg |
| Polyvidon | 115 mg |
| Sorbit | 540 mg |
| Mg-Stearat | 46 mg |
| Citronenaroma | 55 mg |

Das nachfolgende Ausführungsbeispiel 4 beschreibt exemplarisch die erfindungsgemäße Zusammensetzung einer Brausetablette, gemäß eines herkömmlichen Herstellungsprozesses von Brausetabletten, basierend auf einer Direkttablettierung der Wirkstoffe, des Brausesatzes und weiterer Hilfsstoffe.

### Beispiel 4

| | |
|---|---|
| Paracetamol | 500 mg |
| Coffein | 75 mg |
| Chlorphenaminmaleat | 5 mg |
| Vitamin C | 250 mg |
| NaHCO₃ | 800 mg |
| Citronensäure | 1000 mg |
| Sorbit | 540 mg |
| Mg-Stearat | 75 mg |
| Citronenaroma | 55 mg |

Das nachfolgende Ausführungsbeispiele 5 beschreibt exemplarisch die erfindungsgemäße Zusammensetzung eines Brausegranulats, gemäß einem herkömmlichen Herstellungsprozess von Krustengranulaten.

### Beispiel 5

| | |
|---|---|
| Paracetamol | 200 mg |
| Coffein | 75 mg |
| Chlorphenaminmaleat | 2,5 mg |
| Vitamin C | 200 mg |
| Lactose | 600 mg |
| NaHCO3 | 400 mg |
| Citronensäure | 500 mg |
| Orangenaroma | 50 mg |

## Patentansprüche

1. Pharmazeutische Brausezubereitung umfassend einen Brausesatz, bestehend aus einer sauren Komponente und einem basischen CO₂-Spender, eine Wirkstoffkombination und gegebenenfalls weitere Hilfsstoffe und/oder Geschmackskorrigenzien,
**dadurch gekennzeichnet, dass** die Wirkstoffkombination einen Wirkstoff aus der Gruppe der Analgetika, einen Wirkstoff aus der Gruppe der Antihistaminika, Coffein und Ascorbinsäure, umfass, wobei das Antihistaminikum aus Chlorphenamin, Doxylamin Diphenylpyralin und deren Salze und Hydrate ausgewählt ist.

2. Pharmazeutische Brausezubereitung nach Anspruch 1,
**dadurch gekennzeichnet, dass** einer der vier Wirkstoffe in einer Menge unterhalb von 10 mg pro Dosiseinheit enthalten ist.

3. Pharmazeutische Brausezubereitung nach einem der Ansprüche 1 und 2,
**dadurch gekennzeichnet, dass** die Brausezubereitung eine klare Lösung ergibt.

4. Pharmazeutische Brausezubereitung nach einem der Ansprüche 1-3,
**dadurch gekennzeichnet, dass** die Brausezubereitung eine Lösung mit gutem Geschmack ergibt.

5. Pharmazeutische Brausezubereitung nach einem der Ansprüche 2-4,
**dadurch gekennzeichnet, dass** der in einer Menge von weniger als 10 mg enthaltene Wirkstoff homogen in der gelösten Brausezubereitung verteilt ist.

6. Pharmazeutische Brausezubereitung nach einem der Ansprüche 1-5,
**dadurch gekennzeichnet, dass** das Analgetikum aus der Gruppe, bestehend aus Paracetamol, Acetylsalicylsäure, Ibuprofen, Ketoprofen, Meloxicam, Naproxen, Prophyphenazon, Metamizol, sowie deren Salze/Hydrate ausgewählt ist.

7. Pharmazeutische Brausezubereitung nach Anspruch 6, wobei das Analgetikum Paracetamol ist.

8. Pharmazeutische Brausezusammensetzung nach Anspruch 7, wobei die Paracetamolmenge zwischen 200 bis 500 mg pro Einzeldosis beträgt.

9. Pharmazeutische Brausezubereitung nach einem der Ansprüche 1-8,
**dadurch gekennzeichnet, dass** das Antihistaminikum aus Chlorphenaminmaleat, Doxylaminsuccinat und Diphenylpyralin-Hydrochlorid ausgewählt ist.

10. Pharmazeutische Brausezubereitung nach Anspruch 9, wobei das Antihistaminikum Chlorphenaminmaleat ist.

11. Pharmazeutische Brausezusammensetzung nach Anspruch 10, wobei die Chlorphenaminmaleatmenge 2,5 bis 10 mg pro Einzeldosis beträgt.

12. Pharmazeutische Brausezubereitung nach einem der Ansprüche 1-11,
**dadurch gekennzeichnet, dass** die Coffeinmenge 25 bis 75 mg pro Einzeldosis beträgt.

13. Pharmazeutische Brausezubereitung nach einem der Ansprüche 1-12,
**dadurch gekennzeichnet, dass** die Ascorbinmenge 100 bis 300 mg pro Einzeldosis beträgt.

14. Pharmazeutische Brausezubereitung nach einem der Ansprüche 1-13,
**dadurch gekennzeichnet, dass** sie als saure Komponente eine organische Säure, vorzugsweise Citronensäure oder Weinsäure, enthält.

15. Pharmazeutische Brausezubereitung nach einem der Ansprüche 1-14,
**dadurch gekennzeichnet, dass** sie als basischen CO₂-Spender ein Carbonat, Hydrogoncarbonat und/oder Bicarbonat, vorzugsweise Natriumhydrogencarbonat, enthält.

16. Pharmazeutische Brausezubereitung nach einem der Ansprüche 1-15,
**dadurch gekennzeichnet, dass** sie zusätzlich mindestens einen Hilfsstoff, gewählt aus der Gruppe Schmiermittel, Füllstoffe, Bindemittel, Fließreguliermittel, Sprengmittel, Zuckeralkohole, Süßstoffe, Zuckeraustauschstoffe, Aromastoffe enthält.

17. Pharmazeutische Brausezubereitung nach einem der vorangehenden Ansprüche,
**dadurch gekennzeichnet, dass** sie in Form einer Brausetablette vorliegt.

18. Pharmazeutische Brausezubereitung nach einem der vorangehenden Ansprüche,
**dadurch gekennzeichnet, dass** sie in Form eines Brausegranulats vorliegt.

19. Verwendung der pharmazeutischen Brausezubereitung nach einem der vorangehenden Ansprüche zur Herstellung eines Arzneimittels zur Behandlung von Erkältungssymptomen.

## Claims

1. Effervescent pharmaceutical composition comprising an effervescent additive, an active agent combination, and optionally additional pharmaceutical excipients and/or flavoring agents, wherein the effervescent additive consists of an acid component and a basic CO₂ source, **characterized in that** the active agent combination comprises a drug selected from analgetic agents, a drug selected from antihistamines, caffeine and ascorbic acid, wherein the antihistaminic agent is selected from chlorphenamine, doxylamine, diphenylpyraline, and the salts and hydrates thereof.

2. Composition according to claim 1, wherein one of the four active agents is contained in an amount of less than 10 mg per unit dose.

3. Composition according to claim 1 or 2, wherein the composition affords a clear solution.

4. Composition according to any one of claims 1 to 3, wherein the composition affords a solution of good flavor.

5. Composition according to any one of claims 2 to 4, wherein the active agent contained in the composition in an amount of less than 10 mg is homogenously distributed within the dissolved effervescent pharmaceutical composition.

6. Composition according to any one of claims 1 to 5, wherein the analgetic agent is selected from paracetamol, acetylsalicylic acid, ibuprofen, ketoprofen, meloxicame, naproxen, prophyphenazone, metamizole, and the salts/hydrates thereof.

7. Composition according to claim 6, wherein the analgetic agent is paracetamol.

8. Composition according to claim 7, wherein the paracetamol is contained in an amount of between 200 and 500 mg per unit dose.

9. Composition according to any one of claims 1 to 8, wherein the antihistaminic agent is selected from chlorphenamine maleate, doxylamine succinate and diphenylpyraline hydrochloride.

10. Composition according to claim 9, wherein the antihistaminic agent is chlorphenamine maleate.

11. Composition according to claim 10, wherein the chlorphenamine maleate is contained in an amount of between 2.5 and 10 mg per unit dose.

12. Composition according to any one of claims 1 to 11, wherein the caffeine is contained in an amount of between 25 and 75 mg per unit dose.

13. Composition according to any one of claims 1 to 12, wherein the ascorbic acid is contained in an amount of between 100 and 300 mg per unit dose.

14. Composition according to any one of claims 1 to 13, wherein the acid component is an organic acid, preferably citric acid or tartaric acid.

15. Composition according to any one of claims 1 to 14, wherein the basic CO₂ source is a carbonate, hydrogen carbonate and/or bicarbonate, preferably sodium hydrogen carbonate.

16. Composition according to any one of claims 1 to 15, wherein the pharmaceutical excipient is selected from lubricants, fillers, binders, glidants, disintegrants, sugar alcohols, sweeteners, sugar substitutes and flavourings.

17. Composition according to any one of the preceding claims in the form of an effervescent tablet.

18. Composition according to any one of the preceding claims in the form of effervescent granules.

19. Use of an effervescent pharmaceutical composition according to any one of the preceding claims for the preparation of a medicament for the treatment of cold symptoms.

## Revendications

1. Préparation pharmaceutique effervescente comprenant un système effervescent composé d'un composant acide et d'une source alcaline de CO₂, une combinaison de principes actifs et éventuellement d'autres excipients et/ou correcteurs de goût,
**caractérisée en ce que** la combinaison de principes actifs contient un principe actif issu du groupe des analgésiques, un principe actif issu du groupe des antihistaminiques, de la caféine et de l'acide ascorbique, l'antihistaminique étant choisi parmi la chlorphénamine, la doxylamine, la diphénylpyraline et leurs sels et hydrates.

2. Préparation pharmaceutique effervescente selon la revendication 1, **caractérisée en ce que** l'un des quatre principes actifs est présent en quantité inférieure à 10 mg par unité de dose.

3. Préparation pharmaceutique effervescente selon la revendication 1 ou la revendication 2, **caractérisée en ce que** la préparation effervescente produit une solution limpide.

4. Préparation pharmaceutique effervescente selon l'une quelconque des revendications 1 à 3, **caractérisée en ce que** la préparation effervescente produit une solution au goût agréable.

5. Préparation pharmaceutique effervescente selon l'une quelconque des revendications 2 à 4, **caractérisée en ce que** le principe actif présent en quantité inférieure à 10 mg est réparti de manière homogène dans la préparation effervescente dissoute.

6. Préparation pharmaceutique effervescente selon l'une quelconque des revendications 1 à 5, **caractérisée en ce que** l'analgésique est choisi dans le groupe formé par le paracétamol, l'acide acétylsalicylique, l'ibuprofène, le kétoprofène, le méloxicam, le naproxène, la prophyphénazone, le métamizole, ainsi que leurs sels/hydrates.

7. Préparation pharmaceutique effervescente selon la revendication 6, dans laquelle l'analgésique est le paracétamol.

8. Préparation pharmaceutique effervescente selon la revendication 7, dans laquelle la quantité de paracétamol est comprise entre 200 et 500 mg par dose unitaire.

9. Préparation pharmaceutique effervescente selon l'une quelconque des revendications 1 à 8, **caractérisée en ce que** l'antihistaminique est choisi parmi le maléate de chlorphénamine, le succinate de doxylamine et le chlorhydrate de diphénylpyraline.

10. Préparation pharmaceutique effervescente selon la revendication 9, dans laquelle l'antihistaminique est le maléate de chlorphénamine.

11. Préparation pharmaceutique effervescente selon la revendication 10, dans laquelle la quantité de maléate de chlorphénamine est comprise entre 2,5 et 10 mg par dose unitaire.

12. Préparation pharmaceutique effervescente selon l'une quelconque des revendications 1 à 11, **caractérisée en ce que** la quantité de caféine est comprise entre 25 et 75 mg par dose unitaire.

13. Préparation pharmaceutique effervescente selon l'une quelconque des revendications 1 à 12, **caractérisée en ce que** la quantité d'acide ascorbique est comprise entre 100 et 300 mg par dose unitaire.

14. Préparation pharmaceutique effervescente selon l'une quelconque des revendications 1 à 13, **caractérisée en ce qu'**elle comprend comme composant acide un acide organique, de préférence de l'acide citrique ou de l'acide tartrique.

15. Préparation pharmaceutique effervescente selon l'une quelconque des revendications 1 à 14, **caractérisée en ce qu'**elle contient comme source alcaline de CO₂ un carbonate, hydrogénocarbonate et/ou bicarbonate, de préférence de l'hydrogénocarbonate de sodium.

16. Préparation pharmaceutique effervescente selon l'une quelconque des revendications 1 à 15, **caractérisée en ce qu'**elle contient en outre au moins un excipient choisi dans le groupe formé par les lubrifiants, agents de charge, liants, agents d'écoulement, agents désintégrants, polyols, édulcorants, succédanés de sucre, aromatisants.

17. Préparation pharmaceutique effervescente selon l'une quelconque des revendications précédentes, **caractérisée en ce qu'**elle se présente sous la forme d'un comprimé effervescent.

18. Préparation pharmaceutique effervescente selon l'une quelconque des revendications précédentes, **caractérisée en ce qu'**elle se présente sous la forme de granulés effervescents.

19. Utilisation de la préparation pharmaceutique effervescente selon l'une quelconque des revendications précédentes pour produire un médicament destiné à traiter les symptômes du rhume.
